# EUROPEAN PATENT SPECIFICATION

(11) **EP 4 158 356 B1**
(45) Date of publication and mention of the grant of the patent: **10.04.2024**
(21) Application number: 21729553.4
(22) Date of filing: 01.06.2021
(51) Int. Cl.: G01N 33/68

(54) **IN VITRO METHODS FOR THE PROGNOSIS OF AMYOTROPHIC LATERAL SCLEROSIS**
IN-VITRO-VERFAHREN ZUR PROGNOSE VON AMYOTROPHER LATERALSKLEROSE
PROCÉDÉS IN VITRO POUR LE PRONOSTIC DE LA SCLÉROSE LATÉRALE AMYOTROPHIQUE

(30) Priority: 01.06.2020 EP 20382463
(43) Date of publication of application: 05.04.2023
(73) Proprietor: Universidad Del Pais Vasco-Euskal Herriko Unibertsitatea, 48940 Leioa (Bizkaia) (ES); Administración General de la Comunidad Autónoma de Euskadi, 01010 Vitoria-Gasteiz (Araba/Alava) (ES); Fundació Institut d'Investigació Biomèdica de Bellvitge (IDIBELL), 08908 Hospitalet de Llobregat, Barcelona (ES)
(72) Inventor: LÓPEZ DE MUNAIN ARREGUI, Adolfo, 48940 Leioa, Bizkaia (ES); RUIZ-SANZ, José Ignacio, 48940 Leioa, Bizkaia (ES); RUIZ LARREA, Begoña, 48940 Leioa, Bizkaia (ES); FERNÁNDEZ GARCÍA DE EULATE, Gorka, 20014 Donostia-San Sebastian-Gipuzkoa (ES); GEREÑU LOPETEGUI, Gorka, 20014 Donostia-San Sebastian-Gipuzkoa (ES); GIL BEA, Francisco, 20014 Donostia-San Sebastian-Gipuzkoa (ES); POVEDANO PANADÉS, Mònica, 08907 l'Hospitalet de Llobregat (Barcelona) (ES); DOMÍNGUEZ RUBIO, Raúl, 08907 l'Hospitalet de Llobregat (Barcelona) (ES)
(74) Representative: Hoffmann Eitle
(86) International application number: PCT/EP2021/064694
(87) International publication number: WO 2021/245092

(56) References cited:
- WO-A1-2017/037324
- WO-A2-2007/013671
- CHRISTIAN LUNETTA ET AL: "Serum C-Reactive Protein as a Prognostic Biomarker in Amyotrophic Lateral Sclerosis", JAMA NEUROLOGY, vol. 74, no. 6, 1 June 2017 (2017-06-01), pages 660-667, XP055600999, US ISSN: 2168-6149, DOI: 10.1001/jamaneurol.2016.6179
- DORST J ET AL: "Patients with elevated triglyceride and cholesterol serum levels have a prolonged survival in amyotrophic lateral sclerosis", JOURNAL OF NEUROLOGY, STEINKOPFF-VERLAG, DE, vol. 258, no. 4, 3 December 2010 (2010-12-03), pages 613-617, XP019890946, ISSN: 1432-1459, DOI: 10.1007/S00415-010-5805-Z

## Description

### FIELD OF THE INVENTION

The present invention refers to the medical field. Particularly, the present invention refers to an in *vitro* method and kits for the prognosis of amyotrophic lateral sclerosis (ALS).

### STATE OF THE ART

ALS is a rapidly progressive degenerative motor neuron disease for which diagnosis remains based on clinical and electrophysiological findings and the exclusion of other potentially mimicking entities. Although several biochemical, ophthalmological, neurophysiological and imaging biomarkers have been investigated, none of them have been widely introduced in clinical practice. Therefore, there is a pressing need for the identification of simple, accessible and reliable biomarkers for diagnosis and stratification of patients with ALS both in routine medical practice and clinical trials.

Furthermore, the rate of success of clinical trials in ALS patients has been rather poor, and some promising agents in preclinical trials could not be validated in humans. In this line, recent clinical trials for Rasagiline or high-caloric nutrition have failed to find significant benefits on ALS survival, but post hoc analysis demonstrated positive effects in fast-progresing patients with ALS. Thus, prognostic biomarkers may be employed to reduce study subject heterogeneity or target therapeutics to specific subpopulations; increasing statistical power and chances of finding future therapies. A predictive tool, the ENCALS survival model, based on several clinical and paraclinical variables, has proven a reasonable performance. However, there is an unmet need for a robust single biological variable that could aid in stablishing a patient's prognosis and survival at presentation. In this line, although neurofilament light chains have been shown to be significantly elevated in ALS patients when compared to controls, no robust correlation with prognosis and disease progression has been stablished.

The present invention is focused on solving this problem and an *in vitro* method for the prognosis of ALS based on the determination of a specific biomarker is herein provided.

### DESCRIPTION OF THE INVENTION

### Brief description of the invention

Such as it is explained above, the present invention refers to an in *vitro* method and kits for the prognosis of ALS. Particularly, the present invention is focused on building a multivariable prediction models of ALS prognosis using fatty acid (FA) variables.

A prediction model of survival as primary outcome was developed through CoxBoost method modeling from baseline data consisting on 55 serum FA variables and 6 clinical variables previously associated with survival (age, sex, diagnostic delay, type of onset, percentage of theoretical forced vital capacity or %FVC, body mass index) in a longitudinal ALS cohort from Barcelona (n=37). The model was then validated in a dataset from a longitudinal ALS cohort from three different hospitals in Spain (n=38). Secondary outcomes were indication of percutaneous endoscopy gastrostomy (PEG) and/or non-invasive ventilation (NIV). Prognostic performance of models was assessed by inverse probability-of-censoring (IPC) weighting with Uno's C statistic, area under the ROC curve and log-rank p-value, and calibration of models was evaluated by comparing observed and expected survival.

Two clinical variables (age and %FVC) as well as the total ELOVL6 activity were selected as model for ALS survival, which displayed adequate prognostic performance (Uno's C 0.69, 95% CI 0.50-0.82) and well calibration. ELOVL6 was found to be an independent and consistent prognostic value across cohorts for survival (0.62, 0.51-0.77; 0.64, 0.63-0.64) as well as secondary outcomes (PEG and NIV). Finally, alterations of ELOVL6 activity and gene regulation are generalized across different cellular models of ALS.

In conclusion, the present invention reveals that ELOVL6 is an independent predictor of survival in ALS and is robust on different outcomes of ALS staging, showing good generalizability across two independent cohorts, emerging as a biological prognostic biomarker for the development of clinical trials of disease-modifying drugs.

So, the first embodiment of the present invention refers to an *in vitro* method for the prognosis of ALS which comprises: a) measuring the level of expression or activity of ELOVL6, in a biological sample obtained from the subject, and b) wherein if a deviation or variation of the level of expression or activity is identified, as compared with the reference level of expression or activity measured in control patients, this is indicative that the subject has a bad prognosis.

In a preferred embodiment, the present invention refers to an *in vitro* method for the prognosis of ALS which comprises: a) measuring the level of expression or activity of ELOVL6, in a biological sample obtained from the subject, and b) wherein if an overexpression or increased activity is identified, as compared with the reference level of expression or activity measured in control patients, this is indicative that the subject has a bad prognosis.

In a preferred embodiment, the present invention refers to an *in vitro* method for the prognosis of ALS which comprises: a) measuring the level of expression or activity of ELOVL6 in a biological sample obtained from the patient, b) processing the values in order to obtain a risk score and c) wherein if the risk score value measured in the patient is statistically higher as compared with the reference level of expression or activity measured in control patients, this is indicative that the subject has a bad prognosis.

In a preferred embodiment, the biological sample is selected from: serum, plasma or whole blood.

The second embodiment of the present invention refers to the *in vitro* use of ELOVL6 for the prognosis of ALS.

In a preferred embodiment, the present invention refers to the *in vitro* use of ELOVL6 for the prognosis of ALS in a biological sample obtained from the patient selected from: serum, plasma or whole blood.

The third embodiment of the present invention refers to a kit of parts for implementing the *in vitro* method of the invention for the prognosis of ALS which comprises:
a. Reagents or tools for obtaining a biological sample from the patient, and
b. Reagents or tools for determining the level of expression or activity of ELOVL6.

In a preferred embodiment the kit comprises:
a. Reagents or tools for obtaining a biological sample from the patient selected from: serum, plasma or whole blood, and
b. Reagents or tools for determining the level of expression or activity of ELOVL6.

In a preferred embodiment, the present invention refers to the use of the kit for the prognosis of ALS.

The fourth embodiment of the present invention refers to a method for treating or relieving symptoms of patients suffering from ALS which comprises administering an effective amount of a treatment once the survival of the patient is determined by using the above described methods or kits of the invention. Once the patient survival is assessed according to the methodology described in the present invention, any treatment known in the prior art could be used, for instance: riluzole and edaravone.

The fifth embodiment of the present invention refers to a method for the development of clinical trials of disease-modifying drugs once the survival of the patient is determined by using the above described methods or kits of the invention.

The sixth embodiment of the present invention refers to ELOVL6 inhibitors for use in the treatment of amyotrophic lateral sclerosis. The inventors of the present invention demonstrate that the inhibition of ELOVL6 is associated with the therapeutic effect, irrespective of whether the inhibitor inhibits the expression the ELOVL6 gene (such as a shRNA) or it carries out a pharmacological inhibition (such as small molecules).

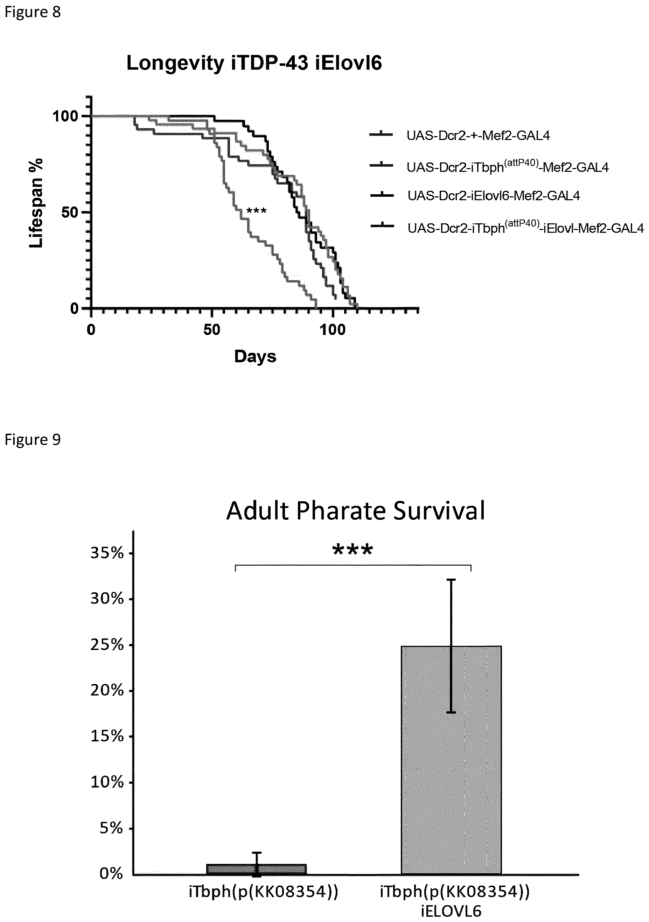

Regarding the inhibitor of the expression the ELOVL6 gene, the present invention shows (see **Example 2.4, Example 2.5,** **Figure 8** and **Figure 9**) a shRNA sequence that targets mRNA of the ELOVL6 fly ortholog gene in *Drosophila* models of TDP-43 knockdown. Thus, other disorders caused by alterations of TDP-43 proteins could be treated following this therapeutic approach like: Spinal muscular atrophy, other pure or complicated motor neuron diseases, Frontotemporal dementias, Alzheimer's disease, Posttraumatic dementia, Neurodegenerative ataxias, Mental Retardation due to Fragile X, autism, VCP myopathy, Inclusion body myositis, DNAJB6 myopathy, inflammatory myopathies, Facioscapulohumeral muscular dystrophies or Myotonic dystrophy.

With respect to the pharmacological inhibition, three families of small molecules can be used: 3-Sulfonyl-8-azabicyclo [3.2.1]octane, Indelodione and Arylcarboxaminde. These molecules can be elected from the following documents which are herein included by reference: WO201/123139, WO2009/131065, WO2008/120653, [Toshiyuki Takahashi, et al., 2009. Synthesis and Evaluation of a Novel Indoledione Class of Long Chain Fatty Acid Elongase 6 (ELOVL6) Inhibitors. J. Med. Chem. 2009, 52, 3142-3145], [Tsuyoshi Nagase, et al., 2009. Synthesis and Biological Evaluation of a Novel3-Sulfonyl-8-azabicyclo[3.2.1]octane Class of Long Chain Fatty Acid Elongase 6 (ELOVL6) Inhibitors. J. Med. Chem. 2009, 52, 4111-4114 4111 DOI: 10.1021/jm900488k]*.*

The seventh embodiment of the present invention refers to an *in vitro* method for monitoring the efficacy of a treatment and/or for predicting the response to a treatment in a patient suffering from amyotrophic lateral sclerosis which comprises: (a) Measuring the level of expression or activity of ELOVL6 in a biological sample isolated from the patient after the treatment; (b) wherein if the level of expression or activity of ELOVL6 determined in step (a) is statistically lower than the level of ELOVL6 before the treatment, it is indicative that the patient is responding to the treatment.

The eight embodiment of the present invention refers to an in vitro method for identifying candidate compounds useful in the treatment of amyotrophic lateral sclerosis which comprises: (a) Measuring the levels of expression or activity of ELOVL6 in a biological sample isolated from the patient after the administration of the candidate compound; (b) wherein if the level of expression or activity of ELOVL6 determined in step (a) is statistically lower than the level of ELOVL6 determined before the administration of the candidate compound, is indicative that the candidate compound is effective in the treatment of amyotrophic lateral sclerosis.

The ninth embodiment of the present invention refers to a method for the determination of a parameter that infers the total activity of ELOVL6, with the objective of implementing any of the methods described in the present invention. Particularly, the following formula can be used: *C14:0*/*C12:0* + *C16:0*/*C14:0* + *C18:0*/*C16:0* + *C18:1n-7*/*C16:1n-7,* wherein the activity of ELOVL6 is calculated by using the levels of six fatty acids: (C12:0, C14:0, C18:0, C16:0, C18:1n-7 y C16:1n-7), being the level of each fatty acid log transformed and the resulting parameter normalized. Following this method, values of total ELOVL6 are ranged between -2 and 2.

For the purpose of the present invention the following terms are defined:
- "Prognostic method" refers to methods used to help predict, at least in part, the course of a disease. For example, this method can be carried out when it is desired to obtain an indication of the future likelihood that the subject will be afflicted with ALS and/or the age at which the subject is likely to develop ALS. In addition, a prognostic method can be carried out on a subject previously diagnosed with ALS or believed or suspected to have ALS, when it is desired to gain greater insight into how the disease will progress for that particular subject (e.g., the likelihood that a particular subject will respond favourably to a particular drug or other treatment, and/or when it is desired to classify or separate ALS patients into distinct and different subpopulations for the purpose of administering a particular type of treatment and/or conducting a clinical trial thereon). A prognostic method can also be used to determine whether and/or how well a subject will respond to a particular drug and/or other treatment.
- The term "comprising" includes, but it is not limited to, whatever follows the word "comprising". Thus, use of the term "comprising" indicates that the listed elements are required or mandatory, but that other elements are optional and may or may not be present.
- By "consisting of" is meant including, and limited to, whatever follows the phrase "consisting of'. Thus, the phrase "consisting of" indicates that the listed elements are required or mandatory, and that no other elements may be present.
- By "effective amount of a treatment" is intended an amount that, when administered as described herein, brings about a positive therapeutic response in the patient, for instance a relief of the symptoms. The exact amount required will vary from subject to subject, depending on (non-exhaustive list): the species, age, general condition of the subject, the severity of the condition being treated or the mode of administration. An appropriate "effective" amount in any individual case may be determined by one of ordinary skill in the art using routine experimentation, based upon the information provided herein.
- The expression "the reference level measured in control patients" refer to a "reference value" of the level of expression or activity of the biomarkers. If a deviation is determined with respect to said "reference", this is an indication of bad prognosis.
- A "reference value" can be a threshold value or a cut-off value. Typically, a "threshold value" or "cut-off value" can be determined experimentally, empirically, or theoretically. A threshold value can also be arbitrarily selected based upon the existing experimental and/or clinical conditions, as would be recognized by a person of ordinary skilled in the art. The threshold value has to be determined in order to obtain the optimal sensitivity and specificity according to the function of the test and the benefit/risk balance (clinical consequences of false positive and false negative). Preferably, the person skilled in the art may compare the biomarker levels (or scores) obtained according to the method of the invention with a defined threshold value. Typically, the optimal sensitivity and specificity (and so the threshold value) can be determined using a Receiver Operating Characteristic (ROC) curve based on experimental data. For example, after determining the levels of the biomarkers in a group of reference, one can use algorithmic analysis for the statistic treatment of the measured concentrations of biomarkers in biological samples to be tested, and thus obtaining a classification standard having significance for sample classification. It is mainly used for clinical biochemical diagnostic tests. ROC curve is a comprehensive indicator that reflects the continuous variables of true positive rate (sensitivity) and false positive rate (1-specificity). It reveals the relationship between sensitivity and specificity with the image composition method. A series of different cut-off values (thresholds or critical values, boundary values between normal and abnormal results of diagnostic test) are set as continuous variables to calculate a series of sensitivity and specificity values. Then, sensitivity is used as the vertical coordinate and specificity is used as the horizontal coordinate to draw a curve. The higher the area under the curve (AUC), the higher the accuracy of diagnosis. On the ROC curve, the point closest to the far upper left of the coordinate diagram is a critical point having both high sensitivity and high specificity values. The AUC value of the ROC curve is between 1.0 and 0.5. When AUC>0.5, the diagnostic result gets better and better as AUC approaches to 1.0. When AUC is between 0.5 and 0.7, the accuracy is low. When AUC is between 0.7 and 0.9, the accuracy is good. When AUC is higher than 0.9, the accuracy is quite high. This algorithmic method is preferably done with a computer. Existing software or systems in the art may be used for the drawing of the ROC curve, such as: MedCalc 9.2.0.1 medical statistical software or SPSS 9.0.

### Description of the figures

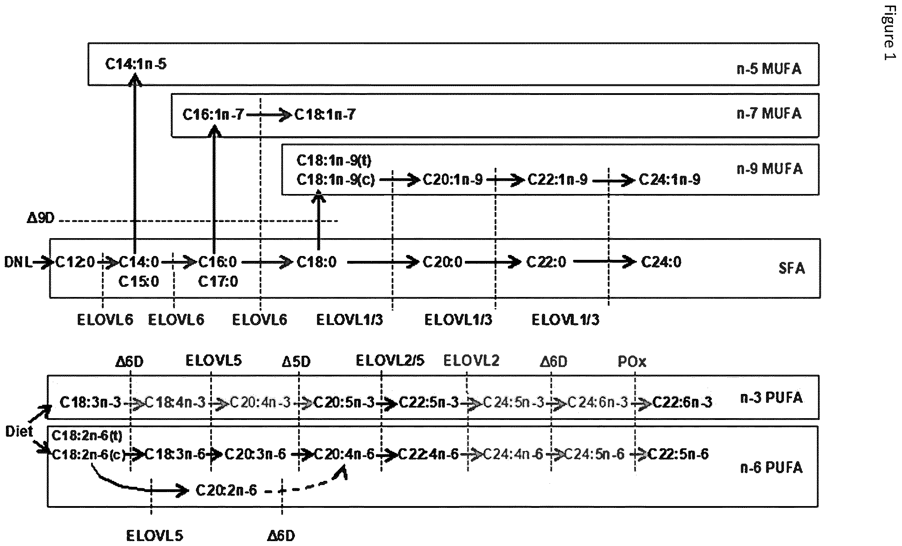

**Figure 1****. FA elongation pathways illustrating the major FAs found in human serum and the enzymatic activities estimated as product-to-precursor ratios.** In dark, variables that were included in the study. In grey, variables not detected or under the detection range. In red, variables selected by CoxBoost method. The variable ELOVL6 selected by the model corresponds to the total ELOVL6 activity, as shown in red. DNL, *de novo* lipid synthesis; Δ5D, delta-5 desaturation by fatty acid desaturase-1 (FADS1); Δ6D, delta-6 desaturation by FADS2; Δ9D, delta-9 desaturation by stearoyl-Coa desaturase-1 (SCD1); ELOVL1-7, elongation of very long-chain fatty acids (1-7); MUFA, monounsaturated fatty acids; SFA, saturated fatty acids; PUFA, polyunsaturated fatty acids.

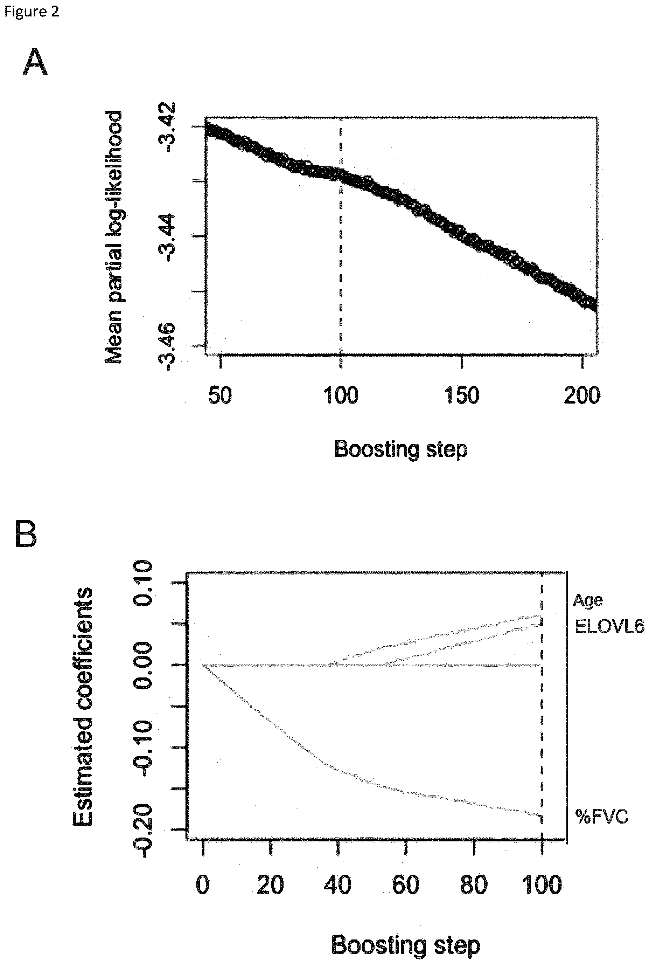

**Figure 2****. Tuning and fitting of CoxBoost model. A,** Number of boosting iterations at which the cross-validated mean partial log-likelihood is optimized (dashed line). Despite partial log-likelihood shows higher values at lower boosting steps, the optimal parameter was chosen at boosting step 100 in order to avoid overfitting and because partial log-likelihood drops considerably after that step. **B,** Selection of variables and estimated coefficients at the optimized partial log-likelihood.

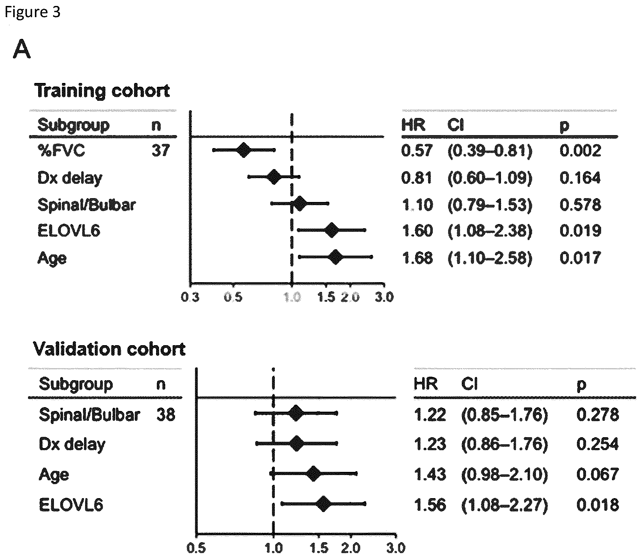

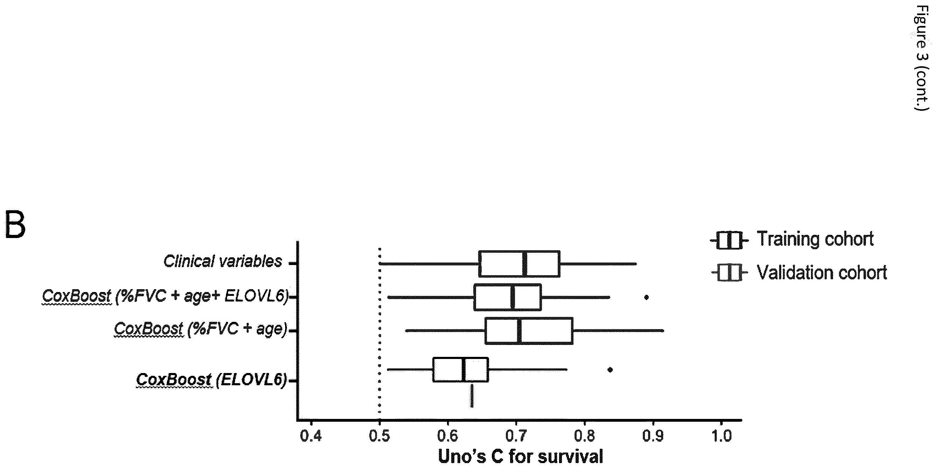

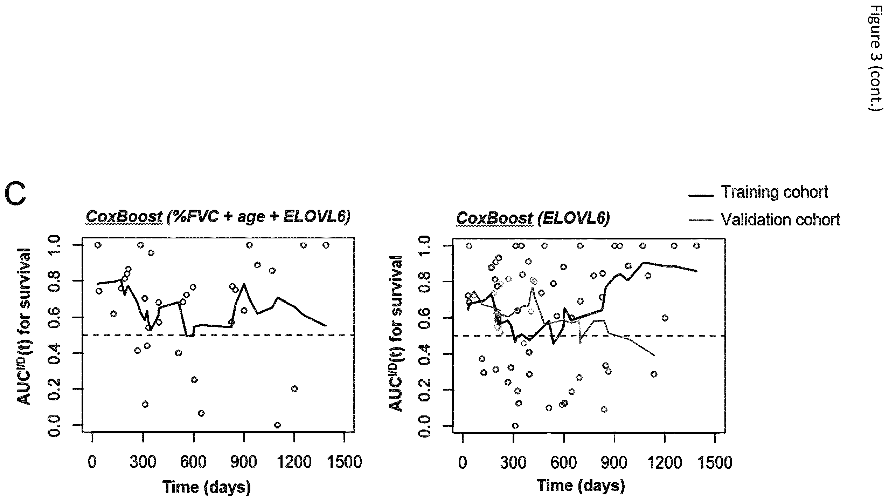

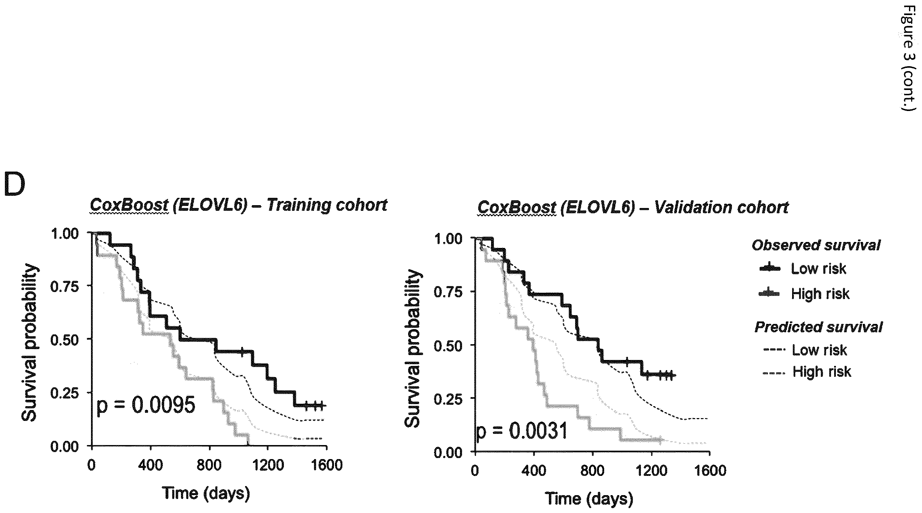

**Figure 3****. ELOVL6 as an independent predictior of ALS survival.** Training dataset is shown in dark blue, validating dataset in orange. A. Univariate Cox proportional-hazards regression analysis for selected variables and other variables comprising the previously developed ENCALS prediction model. **B,** Boxplot of Uno's C statistic for ALS survival comparing the clinical variables that comprise the ENCALS prediction model and the variables selected by the CoxBoost model, with or without the biological parameter ELOVL6. **C,** Representation of time-dependent (incidence/dynamic, I/D) AUC for ALS survival of the three variables selected by CoxBoost model (left panel) and of the biological variable ELOVL6 alone (right panel) on the training (dark blue) and validation cohorts (orange). **D,** Kaplan-Meier plots and log-rank p-values show differences on observed survival between the low-risk or <median (dark blue) and high-risk or ≥median (orange) groups in both the training and validation cohorts. Calibration (concordance of predicted with observed outcome) of the models was assessed by superimposing observed and expected survival predicted by the models (dashed lines).

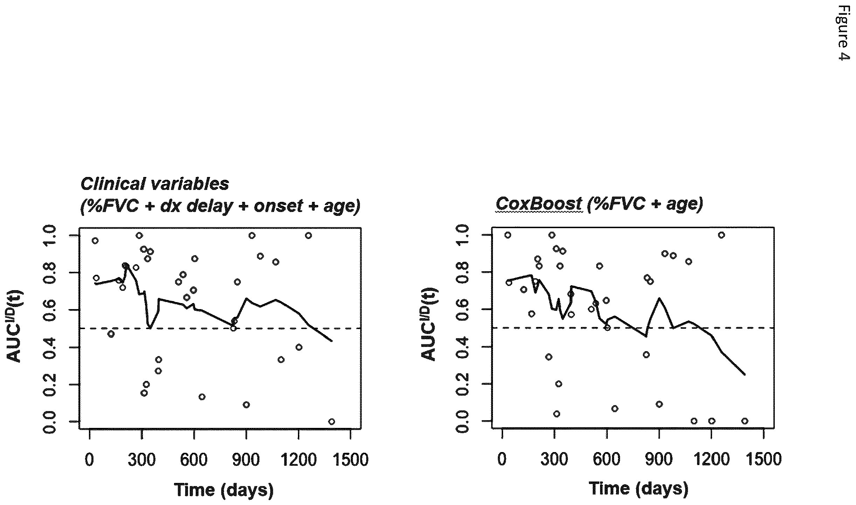

**Figure 4****.** Time-dependent (incidence/dynamic, I/D) AUC values for survival of the clinical variables that comprise the previously developed ENCALS prediction model (left panel) and of the two clinical variables selected by the CoxBoost model (right panel). Prediction scores were calculated by 5-fold cross-validation using CoxBoost method. Both models predict ALS survival within a range of up to one year from sampling but fail to predict longer survival times.

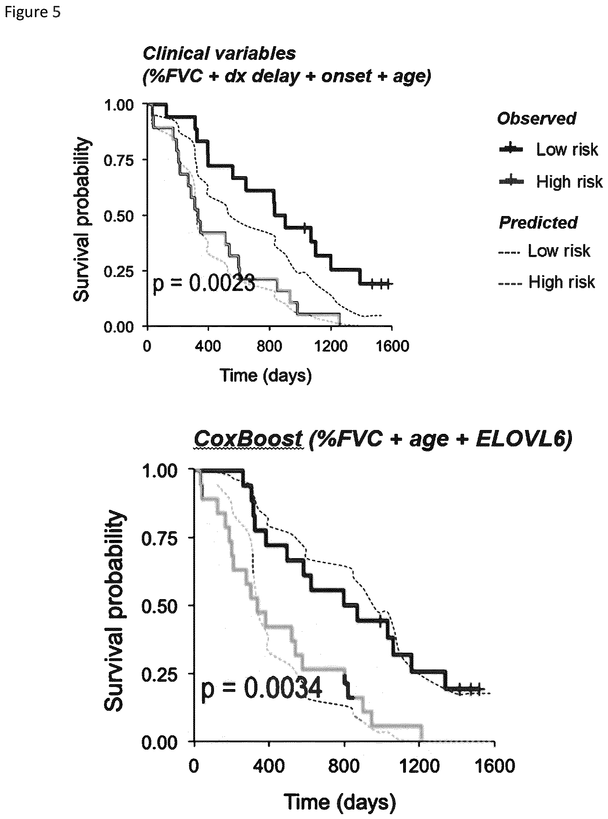

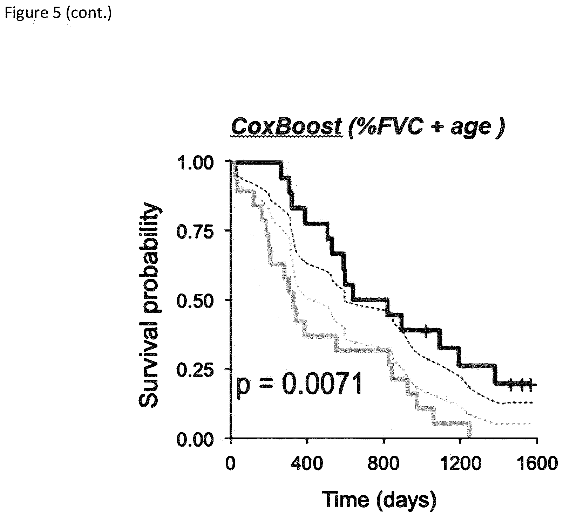

**Figure 5****.** Patients were stratified on two risk groups according to the prediction scores computed by CoxBoost on the clinical variables that comprise the previously developed ENCALS prediction model (top panel) and on the variables selected by the CoxBoost model, with (middle panel) or without the biological variable ELVOL6 (botton panel). Kaplan-Meier plots and log-rank p-values show differences on observed survival between the low-risk (dark blue) and high-risk (orange) groups in the training cohort. Calibration (concordance of predicted with observed outcome) of the models was assessed by superimposing observed and expected survival predicted by the models (dashed lines). The whole CoxBoost model (%FVC, age and ELOVL6) shows the more adequate calibration.

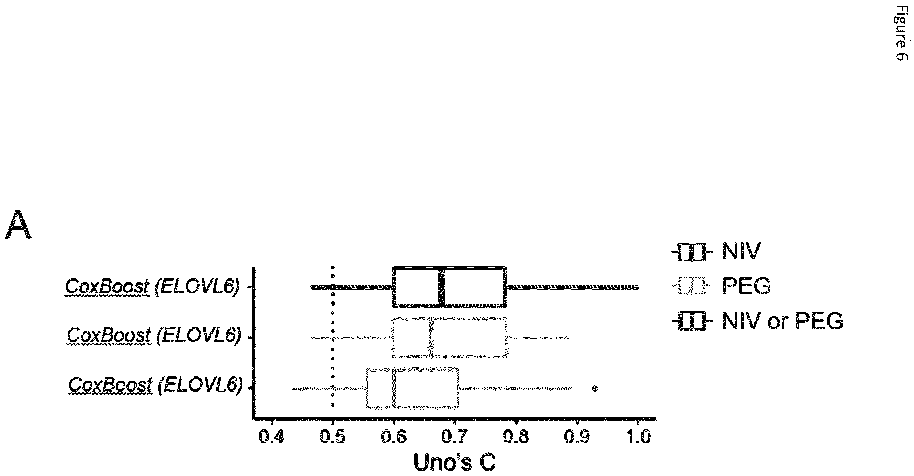

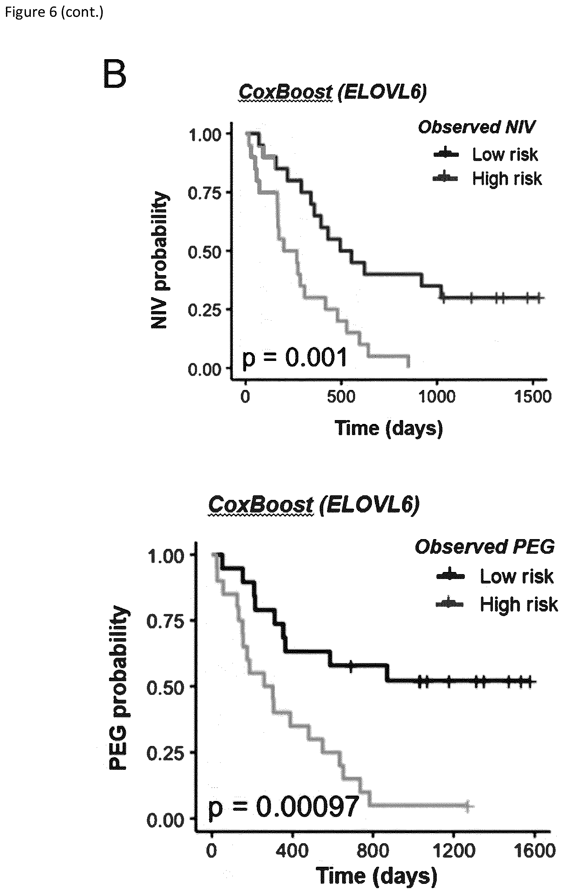

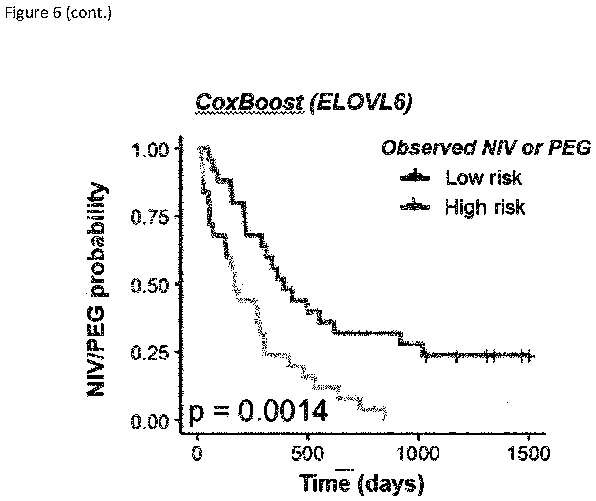

**Figure 6****. ELOVL6 predicts other proxies of ALS developmental staging. A,** Boxplot of Uno's C statistic for the prediction of non-invasive mechanical ventilation (NIV), or percutaneous gastrostomy (PEG) incidences, or both, by ELVOL6 using CoxBoost approach. **B,** Kaplan-Meier plots and log-rank p-values show differences on observed NIV or PEG incidences, or both, between the respective low-risk (dark blue) and high-risk (orange) groups.

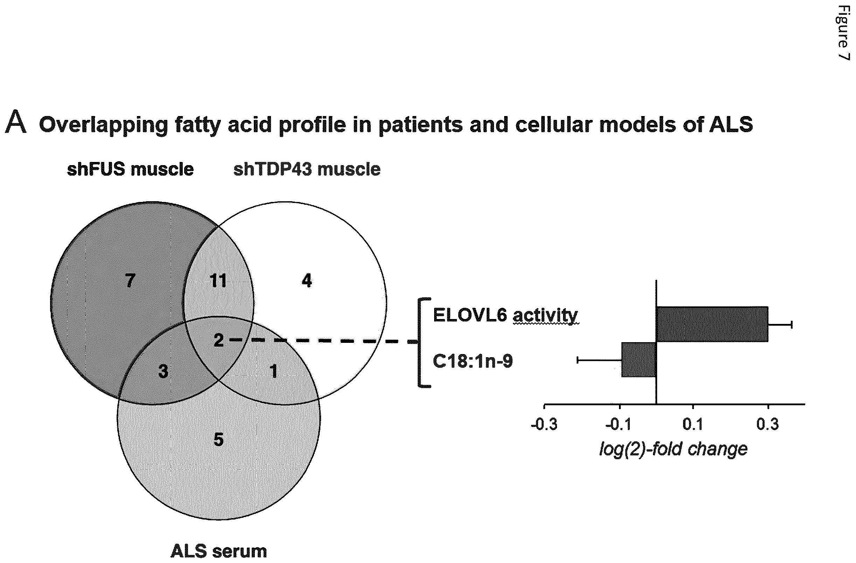

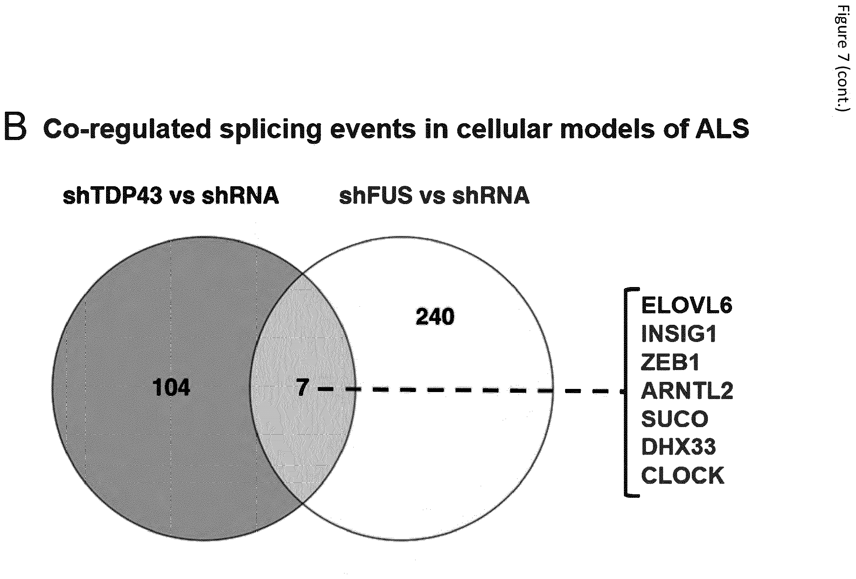

**Figure 7****. Consistent alterations of ELOVL6 in terms of activity and gene regulation in patients and cellular models of ALS.** A, Venn diagram showing overlapping changes in fatty acids between ALS serum and human myoblasts after silencing of TDP-43 or FUS. The activity of ELOVL6, as estimated by total ELOVL6 activity, is the only fatty acid parameter that is strictly coregulated in ALS and in muscle cell models that display dysfunction of ALS genes. Despite changes of C18:1n-9 also overlap, their levels are found increased in ALS serum while decreased in cellular models. **B,** Venn diagram showing coregulated splicing events using Affimetrix RNA microarray data after knockdown of TDP-43 and FUS in human myoblasts. We found 104 TDP43-regulated and 240 FUS-regulated alternative splicing events with significant changes (exon splicing index > 2, exon p-value < 0.01). Among them, only 7 were common splicing events, being two of them genes direclty involved in lipogenic pathways: ELOVL6 and INSIG1.
**Figure 8****. Inhibition of ELVOL6 expression by siRNA extends lifespan in *Drosophila* flies with muscle-conditioned deficiency of TDP-43.** Kaplan-Meyer curves of the control Drosophila strain (UAS-Dicer2-+-Mef2-GAL4, n=45), the Drosophila strain with muscle-conditioned knockdown TDP-43 ortholog gen, Tbph (UAS-Dicer2-iTbph(attP40)-Mef2-GAL4, n=43), Drosophila strain with muscle-conditioned knockdown of ELOVL6 ortholog gene, baldspot (UAS-Dicer2-iElovl6-Mef2-GAL4, n=38), and Drosophila strain with muscle-conditioned knockdown of both Tbph and baldspot (UAS-Dicer2-iTbph(attP40)-iElovl6-Mef2-GAL4, n=43). UAS-Dicer2-iTbph(attP40)-Mef2-GAL4 flies have reduced survival times when compared to control UAS-Dicer2-+-Mef2-GAL4 flies or UAS-Dicer2-iTbph(attP40)-iElovl6-Mef2-GAL4 flies; Mantel-Cox test, ***p<0.001.
**Figure 9****. Inhibition of ELOVL6 expression by siRNA mitigates adult pharate lethality in *Drosophila melanogaster* with muscle-conditioned deficiency of TDP-43.** The Drosophila strain with muscle-conditioned knockdown of TDP-43 ortholog gen, Tbph (UAS-Dicer2-iTbph^{pkk(08354)}-Mef2-GAL4) displays a very prominent reduction in the survival of adult pharate flies. Drosophila strain with muscle-conditioned knockdown of both Tbph and baldspot (UAS-Dicer2-iTbph^{pkk(08354)}-iElovl6-Mef2-GAL4) displays a significant increase in the adult pharate survival when compared to UAS-Dicer2-iTbph^{pkk(08354)}-Mef2-GAL4 flies. Adult pharate survival was calculated by dividing the number of adult flies that emerged from the pupas by the number of total pupas in each tube. The graph shows the average of adult pharate survival of 6 different experiments (n= 77 pupas per group per experiment). T-test was performed; ***p<0,001. Values are expressed as percentage mean ± SD.

### Detailed description of the invention

The present invention is illustrated by means of the examples below which should not be interpreted a limitation in the scope of protection offered by the present invention.

### Example 1. MATERIAL AND METHODS.

### Example 1.1. Study design, participants and data collection.

A training cohort was ascertained selecting patients with suspected motor neuron disease seen at Bellvitge University Hospital between May 17, 2014 and May 2, 2017 (n= 37). Patients i) fulfilled El Escorial criteria of probable or definite ALS (either spinal or bulbar) and ii) blood sampling had been performed before one year from diagnosis of the disease. EMG was performed in all cases to fulfill El Escorial and exclude potential mimics of ALS. Follow-up data were collected until Dec 12, 2019, and the total follow-up time was 78.2 months.

A validating cohort was generated selecting patients fulfilling El Escorial criteria for probable or definite ALS (either spinal or bulbar) from Donostia, Araba, and Marques de Valdecilla University Hospitals in a consecutive manner from Apr 4, 2011 to May 1, 2017 (n=38: 29+4+5 respectively). Data were collected until Jan 10, 2020, and the total follow-up time was 106.8 months.

An interval of at least 2.5 years from sampling was established to assess potential serum lipid biomarkers of ALS survival and progression. This endpoint was established considering the 2-3-year median survival from symptom onset as usually reported [Fogh I, Lin K, Tiloca C, et al. Association of a locus in the CAMTA1 gene with survival in patients with sporadic amyotrophic lateral sclerosis. JAMA Neurol. American Medical Association; 2016; 73:812-820*].* The primary outcome was death, and secondary outcomes were non-invasive ventilation (NIV) and percutaneous endoscopic gastrostomy (PEG) insertion as proxies of King's stage 4 at follow-up (2.5 years). Patients who were still alive at the end of follow-up were censored for the survival analyses. Patients who had NIV or PEG therapy already at the time of sampling were excluded from the assessment of secondary outcomes. As a proportion of patients usually refuse NIV and PEG interventions, we have also excluded those patients who had died with no NIV or PEG therapy in order to avoid confounding factors. Patients who had not received NIV or PEG therapies at the end of the follow-up were censored for the analysis of secondary outcomes.

### Example 1.2. Standard Protocol Approvals, Registrations, and Patient Consents.

The study was approved by the local Ethics Committees of the participant institutions (Ethical Board of Euskadi-Osakidetza, reference number PI2016075, year 2016), and samples were collected after participants gave written informed consent according to the Declaration of Helsinki.

### Example 1.3. Muscle cellular models of ALS.

Immortalized human myoblasts were grown in the proliferation medium consisted of Promocell Skeletal Growth Medium (SGM) supplemented with 10% FBS, 1% Glutamax and 1% gentamicin (both from Gibco-Invitrogen). Gene silencing was carried out by infection with lentiviral particles containing short hairpin RNA (shRNA). The plasmids used to create these particles were: SHC001 (shRNA Empty Vector Control Plasmid DNA), TRCN000016038 (human, TARDBP MISSION shRNA), TRCN0000288639 (human, FUS MISSION shRNA), TRCN00, 33 FOXO MISSION shRNA) (MISSION^{®} pLKO.1-Pure, Sigma-Aldrich). Viral particles were produced and titled by the Viral Vector Unit of CNIC (Madrid). When myoblasts were found at 50% confluence, viral particles at a MOI of 10 were added. After 24 hours the medium was removed and replaced by the fresh SGM proliferation medium. To verify the degree of gene silencing, TDP-43 and FUS proteins were measured by Western Blot (data not shown).

### Example 1.4. Drosophila strains

Two Drosophila strains silencing Tbph gene (ortholog of human TDP-43) at different locus in muscle cells (UAS-Dicer2-iTbph^{(attp40)}-Mef2-GAL4 and UAS-Dicer2-iTbph^{pkk(08354)}-Mef2-GAL4) have been generated. As control flies, the strain UAS-Dicer2-+-Mef2-GAL4 was used. Flies were housed at 24 °C, 70% humidity and 12 h/12 h light/darkness cycle. For longevity assays, 40-100 flies (five per tube) were selected from each strain. Dead flies were counted every 2 days. Kaplan-Meier method was used to plot the results. A Mantel-Cox test was used to analyse survival followed by Bonferroni correction for multiple comparisons.

### Example 1.5. Determinations of serum fatty acids.

### Fatty acid extraction

Lipids were extracted with chloroform/methanol (2:1, v/v) and the chloroform phase was evaporated to dryness under vacuum (Speed-VAC, Savant Inc., Midland, MI, USA). The lipid residue was dissolved in toluene and spotted on thin-layer chromatography plates (SIL G-25, Macherey-Nagel, Duren, Germany). Plates were developed in n-heptane/diisopropyl ether/acetic acid (70:30:2, v/v/v). After spraying the plate with an ethanolic solution of 2',7'-dichlorofluorescein, lipids were made visible under UV light. The bands were scraped, and the fatty acids were transmethylated following Lepage and Roy protocol (Lepage and Roy, 1986).

### Chromatography conditions

The fatty acid methyl esters were analyzed with an HP5890 series II gas chromatograph fitted with a flame ionization detector as previously described in *[*Ruiz-Sanz J-I, Pérez-Ruiz I, Meijide S, Ferrando M, Larreategui Z, Ruiz-Larrea M-B. Lower follicular n-3 polyunsaturated fatty acid levels are associated with a better response to ovarian stimulation. J Assist Reprod Genet [online serial]. Springer New York LLC; 2019; 36: 473-482]*.* Samples were injected through the split injection port (split ratio, 30:1) onto a SP 2330 capillary column (30 m × 0.25 mm, 0.20 mm fi lm thickness; Supelco Company, USA). Helium was used as carrier gas under a pressure of 0.5 bar and injector and detector temperatures were 250 ° C. The oven temperature was programmed at 80° C for 1 min, increased from 80 to 140 ° C at a rate of 50° C/min, then from 140 to 190 ° C at a rate of 5° C/min, and then held at 190° C for 5 min. Finally, the temperature was increased from 190 to 210° C at a rate of 5° C/min and held at 210° C for 15 min. Individual fatty acids were identified by comparing relative retention times with commercial standards (Nu Chek, USA). Heptadecanoic acid was used as an internal standard.

### Example 1.6. RNA microarrays.

The RNA obtained from myoblast cell cultures was analysed using GeneChip complete human transcription expression microarrays of the type Clariom D (Affymetrix). These microarrays analyse more than 540,000 transcripts and allow analysis at two levels: the level of gene expression and the level of alternative splicing. The microarrays were hybridized following the recommended protocols, using a hybridization oven at 45 ° C at 60 rpm for 16 h. After hybridization was completed, a washing and staining step was performed in GeneChip Fluidics Station 450. Image capture was performed on the GeneChip 7G (Affymetrix) scanner. The analysis software used was Expression Console (Affymetrix), Transcriptome Analysis Console (TAC) 4.0 and Gene Set Enrichment Analysis (GSEA; Broad Institute).

### Example 1.7. Statistical analysis.

### Predictor variables

Fifty-five lipid variables were detected, including individual FAs and estimated enzymatic activities as product-to-precursor ratios (**Figure 1**). In addition, six clinical variables were available, including age, sex, type of onset, diagnostic delay, % of forced vital capacity (FVC), and body mass index (BMI) at sampling. Continuous variables were log-transformed and normalized while binary variables were just normalized before being analyzed.

### Outcomes

The primary outcome was survival from sampling. In a secondary analysis, we examined the performance of the selected model by assessing the prediction capacity on two further outcomes as proxies of ALS staging: the need of PEG or NIV interventions, which were defined as the time they were provided.

### Variable selection

For models built from high-dimensional data to predict individual survival probabilities based on the covariates available, the traditional statistical tools cease to be appropriate, thus modern approaches have been developed to perform parameter estimation and selection of covariates simultaneously avoiding discarding covariates before model fitting. One of these approaches is based on boosting, which does so by putting a penalty on the model parameters for estimation. The structure of this penalty is chosen such that most of the estimated parameters will be equal to zero, and therefore the value of the corresponding covariates does not influence predictions obtained from the fitted model.

We have applied a CoxBoost method to fit Cox proportional hazards models based on boosting. In contrast to gradient boosting, CoxBoost is not based on gradients of loss functions, but is based on component wise boosting. It adapts the offset-based boosting approach for estimating Cox proportional hazards models from Tutz and Binder [Tutz G, Binder H. Boosting ridge regression. Comput Stat Data Anal. North-Holland; 2007;51:6044-6059]. In each boosting iteration the previous boosting steps are incorporated as an offset in penalized partial likelihood estimation, which is employed to obtain an update for one single parameter. This results in sparse fits similar to Lasso-like approaches, with many estimated coefficients being zero. Boosting relies on two tuning parameters: penalty and number of boosting steps. The first parameter controls the "weakness" of the estimator, and the second one plays an important role in avoiding overfitting and in controlling the sparsity of the model. We used the R package *CoxBoost,* which exploits the boosting method [*CRAN* - *Package CoxBoost [online]. Accessed at: https:*//*cran.r-project.org*/*web*/*packages*/*CoxBoost*/*index.html. Accessed April 9, 2020].* The optimal number of boosting steps was selected by leave-one-out cross-validation using the *cv.CoxBoost* function applying the penalty value that was chosen by using the *optimCoxBoostPenalty* function. For optimal model performance in likelihood-based boosting, it is suggested that the optimal number of boosting steps should be larger or equal to 50 [Tutz G, Binder H. Generalized additive modelling with implicit variable selection by likelihood based boosting Projektpartner Generalized additive modelling with implicit variable selection by likelihood based boosting. Biometrics [online serial]. International Biometric Society; 2004;401:961-971]*.* Once the tuning parameters were optimized, we obtained the selection of variables and their estimated coefficients by using the *CoxBoost* function.

Sixty-one available covariates, including six clinical variables (type of onset, sex, diagnostic delay, %FVC, age and BMI at sampling) and fifty-five FA parameters (**Figure 1** and **Table 1**) were included in the CoxBoost analysis, with survival from sampling as primary outcome.

**Table 1. Demographic and clinical characteristics of patients with ALS at study in each cohort.**

| | Training cohort | Validating cohort | p-value |
|---|---|---|---|
| Number of patients | 37 | 38 | -- |
| Age at sampling (years)^{a} | 61.6 ± 13.0 | 63.4 ± 12.1 | 0.55^{d} |
| Sex (male/female) | 20/17 | 26/12 | 0.20^{e} |
| Cause (sporadic/familial) | 37/0 | 34/4 | *0.04^{e}* |
| Type of onset (spinal/bulbar) | 28/9 | 33/5 | 0.21^{e} |
| %FVC^{a} | 64.3 ±24.5 | -- | -- |
| ALSFRS-R slope^{a,b,c} | 0.74 ± 0.56 | -- | -- |
| FTD (yes/no) | 4/33 | 2/36 | 0.38^{e} |
| Diagnostic delay (days)^{a} | 404.5 ± 233.3 | 274.0 ± 181.7 | *0.01^{d}* |
| BMI (kg)^{a} | 25.8 ± 3.7 | -- | -- |
| Treatment (statin/antidiabetic) | 7/1 | 5/1 | 0.82^{e} |
| Follow-up (months) | 78.2 | 106.8 | -- |

| | | | |
|---|---|---|---|
| ^{a}Values given as mean ± standard deviation. ^{b}ALSFRS-R slope is given as the decline of score per month and is calculated using the following formula: (48 minus ALSFRS-R score) / (date of ALSFRS-R score minus date of onset). ^{c}ALSFRS-R is available from 20 patients. ^{d}Student's t test. ^{e}Chi-square test. | | | |

### Evaluation of model performance

We used three accuracy parameters to evaluate the performance of the model on both the training and validation cohort. In addition, we used these parameters to compare the prognostic value of the selected variables with the clinical variables that comprise the ENCALS prediction tool (mainly %FVC, diagnostic delay, age at diagnosis and type of onset) [Westeneng H-J, Debray TPA, Visser AE, et al. Prognosis for patients with amyotrophic lateral sclerosis: development and validation of a personalised prediction model. Lancet Neurol [online serial]. Lancet Publishing Group; 2018;17:423-433. Accessed at: http://www.ncbi.nlm.nih.gov/pubmed/29598923. AccessedApril 22, 2020*].*

The first one is the Uno's C statistic, which defines the probability that the predictions for a random pair of subjects are concordant with their outcomes. Uno's C value has the same interpretation as Harrell's concordant index but is based on inverse-probability-of-censoring (IPC) weights to offset the dependence of the Harrell's C on censoring [Uno H, Cai T, Pencina MJ, D Agostino RB, Wei LJ. On the C-statistics for evaluating overall adequacy of risk prediction procedures with censored survival data. Stat Med [online serial]. 2011;30:1105-1117]*.* Thus, this statistic aims to overcome the inaccuracy of the unweighted concordance index when censoring time is correlated with the patient's hazard score. We trained the models on 75% of the randomly selected samples of the training cohort and resampled 50 times on either the remaining 25% holdout test or the whole validation cohort to obtain a prediction score with which we determined the median and 95% CI of Uno's C statistic by using the R package *survAUC.*

The second parameter is the time-dependent (incident/dynamic) area under the ROC curve (AUC). The weighted average of time-specific AUCs is another way to express the concordance index. However, in a descriptive context time-dependent AUC provides a simpler graphical approach and a more global summary than using the concordance index, without having to specify a time interval over which cases accumulate [Heagerty PJ, Zheng Y. Survival model predictive accuracy and ROC curves. Biometrics. 2005;61:92-105]. Furthermore, as an individual's disease status changes over time, and so does his or her prognostic information, such as laboratory measures updated in routine clinic visits, time-dependent AUC is particularly relevant when studying prognosis in diseases that have a high but also heterogeneous rate of progression, such as ALS, where laboratory measures may therefore be determined on heterogeneous baseline times. New concepts and associated statistical methods have been developed to evaluate the time-varying performance of any potential prognostic model, allowing for the estimation of AUC as functions of time, thus providing a detailed estimate of longitudinal model performance for use in practice [Bansal A, Heagerty PJ. A Tutorial on Evaluating the Time-Varying Discrimination Accuracy of Survival Models Used in Dynamic Decision Making. Med. Decis. Mak. SAGE Publications Inc.; 2018. p. 904-916]. In the training cohort, we obtained the prediction score by 5-fold cross-validation by using the CoxBoost method. In the validation cohort, we used the prediction score that was computed by CoxBoost in an exemplary resampling that displayed a median value of Uno's C statistic. The incident/dynamic AUCs were calculated by using the R packages *survivalROC* and *risksetROC,* implemented with the code developed and described by Bansal and Heagerty.

The third parameter is the log-ranked p-value. Patients were stratified on two subgroups of the prediction score computed by CoxBoost: low-risk (below median) and high-risk (above median). A log-ranked p-value was then computed to compare survival distributions estimated by Kaplan-Meier curves between these groups using the R package *survival.* In the training cohort, we obtained the prediction score by 5-fold cross-validation by using the CoxBoost method. In the validation cohort, we used the prediction score that was computed by CoxBoost in an exemplary resampling that displayed a median value of Uno's C statistic. It is worth noticing that the stratification of patients ignores the differences within each subgroup, thus may lead to less accuracy compared to Uno's C statistic or AUC.

### Evaluation of model calibration

Calibration determines the concordance between predicted and observed outcome, which is important for the model to provide probabilistic information. Observed survival distribution between risk subgroups was analyzed by Kaplan-Meier curves as before. Predicted survival probabilities at each given time were computed by CoxBoost. Both the observed survival distributions and the median predicted survival probabilities for each subgroup of patients were plot together to assess their degree of superposition.

### Example 2. RESULTS.

Demographic and clinical characteristics of patients with ALS in each cohort are described in **Table 1.** Of the 37 patients in the training cohort, 5 were censored at the end of follow-up period at Dec 12, 2019 with a median follow-up time of 48.7 months (range 32.4 to 53.6). The remaining 33 patients died during follow-up with a median survival time of 17.0 months from sampling (range 1.0 to 46.4). Of the 38 patients in the validation cohort, 8 were censored at the end of follow-up period at Jan 10, 2020 with a median follow-up time of 45.3 months (range 35.5 to 87.8). The remaining 30 died during follow-up with a median survival time of 13.2 months from sampling (range 1.2 to 37.9).

### Example 2.1. Generation of a prediction model of ALS survival.

55 detected FA variables and 6 clinical variables previously associated with survival (age, sex, type of onset, BMI, %FVC and diagnostic delay) were included in the CoxBoost proportional hazard model (**Table 2**).

**Table 2. Clinical (6) and FA variables (55) included in the CoxBoost proportional hazards method.**

| **Clinical variables** | | | | |
|---|---|---|---|---|
| | Age | Sex | Type of onset | |
| | BMI | %FVC | Diagnostic delay | |

| **Individual fatty acids** | | | | |
|---|---|---|---|---|
| | C12:0 | C14:0 | C14:1n-5 | C15:0 |
| | C16:0 | C16:1n-7 | C17:0 | C18:0 |
| | C18:1n-9(t) | C18:1n-9 | C18:1n-7 | C18:2n-6(t) |
| | C18:2n-6 | C18:3n-6 | C20:0 | C18:3n-3 |
| | C20:1n-9 | C20:2n-6 | C20:3n-6 | C20:4n-6 |
| | C22:1n-9 | CC23:0 | C20:5n-3 | C24:0 |
| | C22:4n-6 | C24:1n-9 | C22:5n-6 | C25:0 |
| | C22:5n-3 | C22:6n-3 | | |

| **Product-to-precursor ratios** | | | | |
|---|---|---|---|---|
| | ELOVL6 (C12:0): | | *C14:0*/*C12:0* | |
| | ELOVL6 (C14:0): | | *C16:0*/*C14:0* | |
| | ELOVL6 (C16:0): | | *C18:0*/*C16:0* | |
| | ELOVL6 (C16:1n-7): | | *C18:1n-7*/*C16:1n-7* | |
| | ELOVL6 (total): | | *C14:0*/*C12:0* + *C16:0*/*C14:0* + *C18:0*/*C16:0* + *C18:1n-7*/*C16:1n-7* | |
| | Δ9D (C14:0): | | *C14:1n-5*/*C14:0* | |
| | Δ9D (C16:0): | | *C16:1n-7*/*C16:0* | |
| | Δ9D (C18:0): | | *C18:1n-9*/*C18:0* | |
| | Δ9D (total): | | *C14:1n-5*/*C14:0* + *C16:1n-7*/*C16:0* + *C18:1n-9*/*C18:0* | |
| | ELOVL3 (C18:1n-9): | | *C20:1n-9*/*C18:1n-9* | |
| | ELOVL3 (C20:1n-9): | | *C22:1n-9*/*C20:1n-9* | |
| | ELOVL3 (C22:1n-9): | | *C24:1n-9*/*C22:1n-9* | |
| | ELOVL3 (total): | | *C20:1n-9*/*C18:1n-9* + *C22:1n-9*/*C20:1n-9* + *C24:1n-9*/*C22:1n-9* | |
| | ELOVL1 (C18:0): | | *C20:0*/*C18:0* | |
| | ELOVL1 (C20:0): | | *C22:0*/*C20:0* | |
| | ELOVL1 (C22:0): | | *C24:0*/*C22:0* | |
| | ELOVL1 (total): | | *C20:0*/*C18:0* + *C22:0*/*C20:0* + *C24:0*/*C22:0* | |
| | Δ6D (C18:2n6): | | *C18:3n-6*/*C18:2n-6* | |
| | Δ5D (C20:3n6): | | *C20:4n-6*/*C20:3n-6* | |
| | ELOVL5 (C18:2n6): | | *C20:2n-6*/*C18:2n-6* | |
| | ELOVL5 (C18:3n6): | | *C20:3n-6*/*C18:3n-6* | |
| | ELOVL5 (C20:5n3): | | *C22:5n-3*/*C20:5n-3* | |
| | ELOVL5 (C20:4n6): | | *C22:4n-6*/*C20:4n-6* | |
| | ELOVL5 (total): | | *C20:2n-6*/*C18:2n-6* + *C20:3n-6*/*C18:3n-6* + *C22:Sn-3*/*C20:Sn-3* + *C22: 4n-6*/*C20: 4n-6* | |
| | Δ8D (C20:2n6): | | *C20:3n-6*/*C20:2n-6* | |

After fitting the CoxBoost model on the training cohort (n=37) via the optimal partial log-likelihood achieved after 100 boosting steps (**Figure 2**), only three variables displayed non-zero coefficients. These were age and %FVC at sampling, and the overall ELVOL6 activity (**Figure 2**) that is calculated from the corresponding products and precursor ratios: C14:0/C12:0, C16:0/C14:0, C18:0/C16:0 and C18:1n-7/C16:1n-7. Each of these variables as well as other variables included in the previously developed ENCALS prediction model (diagnostic delay and type of onset) displayed significant hazard ratios (HR) as it can be observed in the univariate Cox proportional-hazards regression analysis (**Figure 3A**). CoxBoost-estimated coefficients for ELOVL6 and age at sampling were positive and their HRs above 1, meaning that higher values of these variables are associated with shorter survival. Estimated coefficient for %FVC was negative and its HR below 1, meaning that lower values of this variable are associated with shorter survival.

The prognostic performance of the model was evaluated by IPC weighting with Uno's C statistic. Training dataset was randomly split into a 75% training subset and resampled 50 times to calculate the prediction scores using the CoxBoost method. Performance of the models was assessed on the remaining 25% holdout test subset of training dataset and on an external validation cohort of ALS patients from 3 different hospitals (n=38). The selected variables had an Uno's C value of 0.69 (0.50-0.82), which is comparable to the statistic displayed by the clinical variables of the previously developed risk prediction model of ENCALS (0.71, 0.56-0.82) (**Figure 3B**). The difference is that our prediction model includes one biological variable; hence we evaluated the prediction performance of the variable ELOVL6 by itself. It turned out that the Uno's C value of the variable ELOVL6 was slightly decreased when compared to the value of the whole model (0.62, 0.51-0.77), but well reproduced in the validation cohort (0.64, 0.63-0.64) (**Figure 3B**). Harrell's C statistics were also calculated and showed values comparable to Uno's C (data not shown).

By calculating the incidence/dynamic AUC values, we observed that either the selected model or the variable ELOVL6 alone are able to predict ALS survival within a range of up to one year from sampling but fails to predict longer survival times (**Figure 3C**). This result is similar to the
time-dependent AUC for ALS survival yielded by the clinical variables of the previously developed risk prediction model of ENCALS (**Figure 4**).

Patients were stratified in two risk subgroups according to the prediction scores computed by CoxBoost on the variable ELVOL6 in order to compare the survival distribution between the low-risk (<median) and high-risk (≥median) groups. Kaplan-Meier survival plots and log-rank p-values show a significant difference between the low-risk and high-risk groups in both the training (p=0.0095) and validation cohorts (p=0031) (**Figure 3D**). The ELOVL6 model was adequately calibrated (concordance of predicted with observed outcome) in the training cohort; however, survival probability of the high-risk group was infra-estimated in the validation cohort (**Figure 3D**). The whole CoxBoost model, with and without the variable ELOVL6, and the clinical variables of the ENCALS risk prediction model provided differences between the respective low- and high-risk groups with p-values below 0.01 (Kaplan-Meier and calibration plots are shown in **Figure 5**).

### Example 2.2. ELOVL6 predicts other proxies of ALS staging.

The same methodology was carried out to evaluate the ability of ELOVL6 to predicting the time from sampling to the need of PEG or NIV, as proxies of ALS staging. Cases having PEG and/or NIV at sampling, or cases who died without PEG and/or NIV, were excluded from the analysis. The remaining cases from both the training and validation cohorts were combined in a single dataset for this purpose (n=40 for NIV outcome; n=39 for PEG outcome; n=50 for either of the outcomes). As before, the datasets were randomly split into 75% training subsets and resampled 50 times to calculate the prediction scores using the CoxBoost method, with which the remaining 25% holdout subsets were tested. ELOVL6 displayed an Uno's C value of 0.68 (0.49-0.90) for predicting NIV, 0.66 (0.49-0.88) for PEG, and 0.60 (0.49-0.84) for either of both (**Figure 6A**). Patients were again stratified on two risk groups according to the prediction scores computed by CoxBoost on the variable ELOVL6 in order to compare PEG or NIV incidence distribution between the low-risk (<median) and high-risk (≥median) groups. ELOVL6 was associated with the probability of needing NIV support (p=0.001), of undergoing a PEG (p=0.00097) or both (p=0.0014) (**Figure 6B**).

### Example 2.3. Overlapping abnormalities of ELOVL6 in patients and cellular models.

Skeletal muscle is one of the most metabolically active organs in the body, making an important contribution to the circulating metabolome, including in particular lipid species. We hypothesize that FA acid abnormalities detected in serum of patients with ALS may stem from disturbances on muscle function and metabolism in ALS. To test this prediction, we have analyzed the FA profile in a human myoblast cell line that reproduces metabolic and myogenic features of ALS after silencing of either TDP-43 or FUS (data not shown). The advantage of studying an isolated cellular model of skeletal muscle is that any observed change will be secondary to TDP-43 or FUS loss-of-function but independent of the effects of muscle denervation. We found 13 FA features that overlapped after knocking down TDP-43 and FUS. And most strikingly, we observed only 2 FA features that were consistently altered in the cellular models and in serum samples from patients with ALS relative to controls. These FA features were C18:1n-9 and the total ELOVL6 activity (**Figure 7A**). However, only ELOVL6 displayed changes in the same direction in the different groups.

Since TDP-43 and FUS are RNA-binding proteins with important roles in regulating RNA splicing, we studied alternative splicing events after knockdown of TDP-43 and FUS in human myoblasts by using Affymetrix Clariom^{™} D expression microarrays. We detected 240 and 104 significant alternative splicing events (exon splicing index > 2, p-value < 0.01) in FUS- and TDP-43-silenced cells. Among the 7 co-regulated alternative splicing events in both conditions, ELOVL6 and another gene involved in lipogenic pathways, INSIG1, were found (**Figure 7B**).

Overall, these results suggest that ELOVL6 may be a common pathogenic target of ALS mediating FUS and TDP-43 loss-of-function toxicity in skeletal muscle. ELOVL6 might be a modifier of disease, and that would explain why its reflected activity in blood is an indicator of ALS disease prognosis.

### Example 2.4. siRNA that targets mRNA of the ELOVL6 fly ortholog gene baldspot extends lifespan in Drosophila flies with muscle-conditioned deficiency of TDP-43.

We generated a *Drosophila* model in which *Tbph* gene, which is ortholog of human TDP-43, was conditionally silenced in muscle (*iTbph^{attp40}*) by a siRNA of SEQ ID NO: 1 (CCAGTTAAGAATTGTATTTAA) targeting that gene. We also generated a second *Drosophila* model in which *baldspot* gene, which is ortholog of human *ELOVL6,* was conditionally silenced in muscle (*iElovl6*) by a siRNA of SEQ ID NO: 2 (CTGGACATGGGTGTTCTACTA) targeting that targets that gene, and a third *Drosphila* model in which both *baldspot* and *Tbph* genes were conditionally silenced in muscle (*iTbph^{attp40}-iElovl6*).

We performed longevity assays to characterize these fly models. *Tbph*-silenced flies manifested dramatic reductions of survival times when compared to control group, as indicated by Kaplan-Meier curves (**Figure 8**). Interestingly, flies with double knockdown of *Tbph* and *baldspot* (ELOVL6 ortholog) had higher survival than flies with only *Tbph* knockdown (**Figure 8**). It is important to note that *baldspot* konckdown does not affect life span in control flies (**Figure 8**), thereby suggesting lack of toxicity when inhibiting ELOVL6 activity *in vivo.*

### Example 2.5. siRNA that targets mRNA of the ELOVL6 fly ortholog gene baldspot mitigates adult pharate lethality in Drosophila melanogaster with muscle-conditioned deficiency of TDP-43.

We generated a *Drosophila* model in which *Tbph* gene, which is ortholog of human TDP-43, was conditionally silenced in muscle (*iTbph^{pkk(08354)}*) by a siRNA of SEQ ID NO: 3 (CTCCGCAGATCCAGCATTATAAGTGCACAATCTTTATAACTAAACTTTCTTAGCG TAAGCCAAAATATATTTAATAAACAACAAAATACAAATCAAATTCTACTTAAGTG ATACAATGATACAATTTTTACTGAGCTTTGGCGCAGAATTTATTTTGAAGTGTGTT TTATCAATTGTGTTATTAGCCATCCAGTGTCATCTTGTTTACAATTATCAAAATAT CTATTACATATTAAATACAATTCTTAACTGGTTTGTATGTGGAATACATATATGTA AATTTAAGTAAGCTACAGAGGTGTTGGTTAACCACATTAACCACATCATTGGGTG ACAGGCACCGCCTTAAAGAAAGTTTGACTTCTCCGCGGCGTTTTGAGATCCGCTG CTCT) targeting that gene. Flies of this genotype completed the metamorphosis from larvae to adult within the pupa (a phase known as pharate) but were not able to emerge from the pupa and, consequently, died. This feature is termed as adult pharate lethality and allowed us to perform assays with which to judge the efficacy of ELOVL6 inhibition. We also generated a *Drosophila* model in which *baldspot* gene, which is ortholog of human *ELOVL6,* was conditionally silenced in muscle of iTbph^{pkk(108254)} flies by siRNA of SEQ ID NO: 2 (CTGGACATGGGTGTTCTACTA) targeting this gene (*iElovl6*). By measuring this outcome we observed that targeting *baldspot* (ELOVL6 ortholog) mRNA in the i*Tbph^{pkk(108254)}* Drosophila model significantly mitigated pharate lethality by approximately 30% (**Figure 9**).

## Claims

1. *In vitro* method for the prognosis of amyotrophic lateral sclerosis which comprises: a) measuring the level of expression or activity of ELOVL6, in a biological sample obtained from the subject, and b) wherein if a deviation or variation of the level of expression or activity is identified, as compared with the reference level of expression or activity measured in control patients, this is indicative that the subject has a bad prognosis.

2. *In vitro* method, according to claim 1, which comprises: a) measuring the level of expression or activity of ELOVL6, in a biological sample obtained from the subject, and b) wherein if an overexpression or increased activity is identified, as compared with the reference level of expression or activity measured in control patients, this is indicative that the subject has a bad prognosis.

3. *In vitro* method, according to any of the previous claims, which comprises a) measuring the level of expression or activity of ELOVL6 in a biological sample obtained from the patient, b) processing the values in order to obtain a risk score and c) wherein if the risk score value measured in the patient is statistically higher as compared with the reference level of expression or activity measured in control patients, this is indicative that the subject has a bad prognosis.

4. *In vitro* method, according to any of the claims 1 to 3, wherein the biological sample is selected from: serum, plasma or whole blood.

5. *In vitro* use of ELOVL6 for the prognosis of amyotrophic lateral sclerosis.

6. *In vitro* use of ELOVL6, according to claim 5, for the prognosis of amyotrophic lateral sclerosis in a biological sample obtained from the patient selected from: serum, plasma or whole blood.

7. Use of a kit which comprises:
a. Reagents or tools for obtaining a biological sample from the patient, and
b. Reagents or tools for determining the level of expression or activity of ELOVL6,
for the prognosis of amyotrophic lateral sclerosis.

8. Use of a kit, according to claim 7, which comprises:
a. Reagents or tools for obtaining a biological sample from the patient selected from: serum, plasma or whole blood, and
b. Reagents or tools for determining the level of expression or activity of ELOVL6,
for the prognosis of amyotrophic lateral sclerosis.

9. ELOVL6 inhibitor for use in the treatment of amyotrophic lateral sclerosis.

10. ELOVL6 inhibitor for use, according to claim 9, wherein the inhibitor inhibits the expression the ELOVL6 gene or it carries out a pharmacological inhibition.

11. ELOVL6 inhibitor for use, according to any of the claims 9 or 10, wherein the inhibitor is a siRNA **characterized by** the sequence SEQ ID NO: 1, 2 or 3.

12. *In vitro* method for monitoring the efficacy of a treatment and/or for predicting the response to a treatment in a patient suffering from amyotrophic lateral sclerosis which comprises: (a) Measuring the level of expression or activity of ELOVL6 in a biological sample isolated from the patient after the treatment; (b) wherein if the level of expression or activity of ELOVL6 determined in step (a) is statistically lower than the level of ELOVL6 before the treatment, it is indicative that the patient is responding to the treatment.

13. *In vitro* method for identifying candidate compounds useful in the treatment of amyotrophic lateral sclerosis which comprises: (a) Measuring the level of expression or activity of ELOVL6 in a biological sample isolated from the patient after the administration of the candidate compound; (b) wherein if the level of expression or activity of ELOVL6 determined in step (a) is statistically lower than the level of ELOVL6 determined before the administration of the candidate compound, is indicative that the candidate compound is effective in the treatment of amyotrophic lateral sclerosis.

14. *In vitro* method, according to any of the claims 12 or 13, wherein the biological sample is selected from: serum, plasma or whole blood.

## Patentansprüche

1. *In-vitro-*Verfahren zur Prognose von amyotropher Lateralsklerose, welches Folgendes umfasst: a) das Messen des Expressionsniveaus oder der Aktivität von ELOVL6, in einer aus dem Individuum erhaltenen biologischen Probe, und b) wobei, wenn eine Abweichung oder Variation des Expressionsniveaus oder der Aktivität identifiziert wird, im Vergleich zum Referenz-Expressionsniveau oder zur Referenz-Aktivität, welches/welche in Kontrollpatienten gemessen wurde, dies darauf schließen lässt, dass das Individuum eine schlechte Prognose hat.

2. *In-vitro*-Verfahren nach Anspruch 1, welches Folgendes umfasst: a) das Messen des Expressionsniveaus oder der Aktivität von ELOVL6, in einer aus dem Individuum erhaltenen biologischen Probe, und b) wobei, wenn eine Überexpression oder erhöhte Aktivität identifiziert wird, im Vergleich zum Referenz-Expressionsniveau oder zur Referenz-Aktivität, welches/welche in Kontrollpatienten gemessen wurde, dies darauf schließen lässt, dass das Individuum eine schlechte Prognose hat.

3. *In-vitro-*Verfahren nach einem der vorhergehenden Ansprüche, welches Folgendes umfasst: a) das Messen des Expressionsniveaus oder der Aktivität von ELOVL6 in einer aus dem Patienten erhaltenen biologischen Probe, b) das Verarbeiten der Werte, um eine Risikoeinstufung zu erhalten, und c) wobei, wenn der Wert der Risikoeinstufung, welcher im Patienten gemessen wurde, statistisch höher im Vergleich zum Referenz-Expressionsniveau oder zur Referenz-Aktivität, welches/welche in Kontrollpatienten gemessen wurde, ist, dies darauf schließen lässt, dass das Individuum eine schlechte Prognose hat.

4. *In-vitro-*Verfahren nach einem der Ansprüche 1 bis 3, wobei die biologische Probe aus Folgenden ausgewählt wird: Serum, Plasma oder Vollblut.

5. *In-vitro*-Verwendung von ELOVL6 zur Prognose von amyotropher Lateralsklerose.

6. *In-vitro*-Verwendung von ELOVL6 nach Anspruch 5, zur Prognose von amyotropher Lateralsklerose in einer aus dem Patienten erhaltenen biologischen Probe, ausgewählt aus: Serum, Plasma oder Vollblut.

7. Verwendung eines Kits, welches Folgendes umfasst:
a. Reagenzien oder Werkzeuge zum Erhalten einer biologischen Probe aus dem Patienten, und
b. Reagenzien oder Werkzeuge zum Bestimmen des Expressionsniveaus oder der Aktivität von ELOVL6,
zur Prognose von amyotropher Lateralsklerose.

8. Verwendung eines Kits nach Anspruch 7, welches Folgendes umfasst:
a. Reagenzien oder Werkzeuge zum Erhalten einer biologischen Probe aus dem Patienten, ausgewählt aus: Serum, Plasma oder Vollblut, und
b. Reagenzien oder Werkzeuge zum Bestimmen des Expressionsniveaus oder der Aktivität von ELOVL6,
zur Prognose von amyotropher Lateralsklerose.

9. ELOVL6-Inhibitor für dessen Verwendung bei der Behandlung von amyotropher Lateralsklerose.

10. ELOVL6-Inhibitor für dessen Verwendung nach Anspruch 9, wobei der Inhibitor die Expression des ELOVL6-Gens hemmt oder eine pharmakologische Inhibierung durchführt.

11. ELOVL6-Inhibitor für dessen Verwendung nach einem der Ansprüche 9 oder 10, wobei der Inhibitor eine siRNS ist, welche durch die Sequenz SEQ ID NO: 1, 2 oder 3 gekennzeichnet ist.

12. *In-vitro*-Verfahren zum Überwachen der Wirksamkeit einer Behandlung und/oder zum Vorhersagen der Antwort auf eine Behandlung in einem Patienten, welcher unter amyotropher Lateralsklerose leidet, welches Folgendes umfasst: (a) das Messen des Expressionsniveaus oder der Aktivität von ELOVL6 in einer biologischen Probe, welche aus dem Patienten nach der Behandlung isoliert wurde; (b) wobei, wenn das Expressionsniveau oder die Aktivität von ELOVL6, welches/welche in Schritt (a) bestimmt wurde, statistisch niedriger als das Niveau von ELOVL6 vor der Behandlung ist, dies darauf schließen lässt, dass der Patient auf die Behandlung antwortet.

13. *In-vitro*-Verfahren zum Identifizieren von Kandidatverbindungen, welche bei der Behandlung von amyotropher Lateralsklerose nützlich sind, welches Folgendes umfasst: (a) das Messen des Expressionsniveaus oder der Aktivität von ELOVL6 in einer biologischen Probe, welche aus dem Patienten nach der Verabreichung der Kandidatverbindung isoliert wurde; (b) wobei, wenn das Expressionsniveau oder die Aktivität von ELOVL6, welches/welche in Schritt (a) bestimmt wurde, statistisch niedriger als das Niveau von ELOVL6, welches vor der Verabreichung der Kandidatverbindung bestimmt wurde, ist, dies darauf schließen lässt, dass die Kandidatverbindung bei der Behandlung von amyotropher Lateralsklerose wirksam ist.

14. *In-vitro*-Verfahren nach einem der Ansprüche 12 oder 13, wobei die biologische Probe aus Folgenden ausgewählt wird: Serum, Plasma oder Vollblut.

## Revendications

1. Procédé *in vitro* pour le pronostic de la sclérose latérale amyotrophique qui comprend : a) mesurer le niveau d'expression ou d'activité d'ELOVL6, dans un échantillon biologique obtenu du sujet, et b) dans lequel si une déviation ou variation du niveau d'expression ou d'activité est identifiée, en comparaison avec le niveau de référence d'expression ou d'activité mesuré chez des patients témoins, cela est une indication que le sujet a un mauvais pronostic.

2. Procédé *in vitro,* selon la revendication **1**, qui comprend : a) mesurer le niveau d'expression ou d'activité d'ELOVL6, dans un échantillon biologique obtenu du sujet, et b) dans lequel si une surexpression ou une activité accrue est identifiée, en comparaison avec le niveau de référence d'expression ou d'activité mesuré chez des patients témoins, cela est révélateur que le sujet a un mauvais pronostic.

3. Procédé in vitro, selon l'une quelconque des revendications précédentes, qui comprend a) mesurer le niveau d'expression ou d'activité d'ELOVL6 dans un échantillon biologique obtenu du patient, b) traiter les valeurs afin d'obtenir un score de risque et c) dans lequel si la valeur du score de risque mesurée chez le patient est statistiquement plus haute en comparaison avec le niveau de référence d'expression ou d'activité mesuré chez des patients témoins, cela est révélateur que le sujet a un mauvais pronostic.

4. Procédé *in vitro* selon l'une des revendications 1 à 3, dans lequel l'échantillon biologique est sélectionné à partir de sérum, de plasma ou de sang total.

5. Utilisation *in vitro* d'ELOVL6 pour le pronostic de la sclérose latérale amyotrophique.

6. Utilisation *in vitro* d'ELOVL6, selon la revendication 5, pour le pronostic de la sclérose latérale amyotrophique dans un échantillon biologique obtenu du patient sélectionné à partir de : sérum, plasma ou sang total.

7. Utilisation d'un kit qui comprend :
a. des réactifs ou des outils pour obtenir un échantillon biologique du patient, et
b. des réactifs ou des outils pour déterminer le niveau d'expression ou d'activité d'ELOVL6,
pour le pronostic de la sclérose latérale amyotrophique.

8. Utilisation d'un kit, selon la revendication 7, qui comprend :
a. des réactifs ou des outils pour obtenir un échantillon biologique du patient sélectionné à partir de : sérum, plasma ou sang total, et
b. des réactifs ou des outils pour déterminer le niveau d'expression ou d'activité d'ELOVL6,
pour le pronostic de la sclérose latérale amyotrophique.

9. Inhibiteur d'ELOVL6 pour utilisation dans le traitement de la sclérose latérale amyotrophique.

10. Inhibiteur d'ELOVL6 pour utilisation, selon la revendication 9, dans lequel l'inhibiteur inhibe l'expression du gène ELOVL6 ou réalise une inhibition pharmacologique.

11. Inhibiteur d'ELOVL6 pour utilisation, selon l'une quelconque des revendications 9 ou 10, dans lequel l'inhibiteur est un pARNi **caractérisé par** la séquence SEQ ID NO : 1, 2 ou 3.

12. Procédé *in vitro* pour contrôler l'efficacité d'un traitement et/ou pour prédire la réponse à un traitement chez un patient souffrant de sclérose latérale amyotrophique qui comprend : (a) mesurer le niveau d'expression ou d'activité d'ELOVL6 dans un échantillon biologique isolé du patient après le traitement ; (b) dans lequel si le niveau d'expression ou d'activité d'ELOVL6 déterminé à l'étape (a) est statistiquement plus bas que le niveau d'ELOVL6 avant le traitement, cela est révélateur que le patient répond au traitement.

13. Procédé *in vitro* pour l'identification de composés candidats utiles dans le traitement de la sclérose latérale amyotrophique qui comprend : (a) mesurer le niveau d'expression ou d'activité d'ELOVL6 dans un échantillon biologique isolé du patient après l'administration du composé candidat ; (b) dans lequel si le niveau d'expression ou d'activité d'ELOVL6 déterminé à l'étape (a) est statistiquement plus bas que le niveau d'ELOVL6 déterminé avant l'administration du composé candidat, cela est révélateur que le composé candidat est efficace dans le traitement de la sclérose latérale amyotrophique.

14. Procédé *in vitro* selon l'une quelconque des revendications 12 ou 13, dans lequel l'échantillon biologique est sélectionné à partir de : sérum, plasma ou sang total.
